# EUROPÄISCHE PATENTSCHRIFT

(11) **EP 4 056 272 B1**
(45) Veröffentlichungstag und Bekanntmachung des Hinweises auf die Patenterteilung: **26.11.2025**
(21) Anmeldenummer: 21162090.1
(22) Anmeldetag: 11.03.2021
(51) Int. Cl.: B01L 3/00, C12M 1/32, C12M 1/00, C12M 1/12

(54) **VORRICHTUNG ZUM NIEDERHALTEN VON JEWEILIGEN MEMBRANELEMENTEN IN JEWEILIGEN VERTIEFUNGEN EINER MULTI-WELL-PLATTE**
DEVICE FOR HOLDING DOWN MEMBRANE ELEMENTS IN RECESSES OF A MULTI-CORRUGATED PLATE
DISPOSITIF DE MAINTIEN PROCHE DES ÉLÉMENTS MEMBRANES RESPECTIFS DANS LES CAVITÉS RESPECTIVES D'UNE PLAQUE À PUITS MULTIPLES

(43) Veröffentlichungstag der Anmeldung: 14.09.2022
(73) Patentinhaber: EUROIMMUN Medizinische Labordiagnostika AG, 23560 Lübeck (DE)
(72) Erfinder: HUTH, Bianca, 23560 Lübeck (DE); AUER, Patrick, 23560 Lübeck (DE); ROSENSTOCK, Philip, 23560 Lübeck (DE)

(56) Entgegenhaltungen:
- US-A- 5 474 742
- US-A1- 2007 082 390
- US-A1- 2010 190 197
- US-A1- 2013 260 410

## Beschreibung

Aus dem Stand der Technik sind sogenannte Multi-Well-Platten bekannt, welche mehrere Vertiefungen aufweisen, wobei eine jeweilige Vertiefung eine Öffnung an der Oberseite der Multi-Well-Platte aufweist. Diese Vertiefungen werden auch Wells genannt. Die jeweiligen Vertiefungen sind jeweils zur Aufnahme einer jeweiligen flüssigen Lösung ausgebildet. Ferner ist es bekannt, dass innerhalb einer solchen Vertiefung neben der flüssigen Lösung wenigstens ein Membranelement in die Vertiefung bzw. in die flüssige Lösung eingelegt werden kann.

Ein solches Membranelement weist vorzugsweise getrocknete Blutbestandteile einer Patientenprobe auf. Für den Zweck des Eluierens bzw. des Lösens von solchen getrockneten Blutbestandteilen aus dem Membranelement wird das Membranelement in die Lösung für einige Zeit eingebracht bzw. eingelegt. Eine solche Lösung kann vorzugsweise eine wässrige Lösung wie insbesondere ein sogenannter Puffer sein. Ein solches Eluieren bzw. Lösen von getrockneten Blutbestandteilen aus dem Membranelement erfolgt zu dem Zweck, dass ein in den getrockneten Blutbestandteilen vorhandener Analyt in die Lösung überführt wird.

Insbesondere ist das Membranelement aus einem absorptionsfähigen Membranmaterial. Ein solches Membranelement wird beispielsweise zuvor aus einer sogenannten Dried-Blood-Spot-Karte herausgestanzt bzw. herausgetrennt, welches dann einen sogenannten "Dried-Blood-Spot" (DBS) ergibt. Das Patientenblut wurde zuvor beispielsweise als Kapillarblut auf den entsprechenden Bereich des Membranelementes auf der Dried-Blood-Spot-Karte aufgebracht. Dies kann beispielsweise durch Anstechen einer Fingerspitze mit einer Lanzette erfolgen, sodass der Patient dann Kapillarblut aus der Fingerspitze heraus auf den entsprechenden Bereich des Membranelementes der Dried-Blood-Spot-Karte auftropfen kann.

Im Zuge einer Prozessierung bzw. eines Verfahrens zum Zwecke des Eluierens bzw. Lösens der getrockneten Blutbestandteile aus dem Membranelement mittels der Lösung kann nach einer ersten Standzeit die Multi-Well-Platte in vorzugsweise abgedecktem Zustand auf einem sogenannten Rotationsschüttler positioniert werden, auf welchem die Multi-Well-Platte dann für einige Zeit einer Schüttelbewegung unterzogen wird. Mittels einer Bewegung des Membranelementes in der Lösung kann hierdurch vorzugsweise die Extraktion bzw. das Eluieren / das Lösen der getrockneten Blutbestandteile aus dem Membranelement heraus unterstützt werden. Anschließend kann dann aus einer Vertiefung mittels eine Pipettiernadel bzw. Aspirationsnadel zumindest ein Teilvolumen der Flüssigkeit mit darin gelösten Blutbestandteilen aspiriert und in ein separates Gefäß überführt werden. In dem Gefäß kann dann mittels weiterer Schritte eines Analyseverfahrens ein möglicherweise vorhandener Analyt nachgewiesen werden. Bei der Aspiration des Teilvolumens der Flüssigkeit aus der Vertiefung kann es hierbei möglicherweise zu einem unerwünschten Verstopfen der Nadel kommen, so dass eine nicht korrekt Menge des Teilvolumens der Flüssigkeit aspiriert wird und ein Prozessierungsergebnis des Analyseverfahrens möglicherweise verfälscht wird.

Für viele qualitative Bestimmungen auf dem Gebiet der Analytik und speziell der Labordiagnostik kommt es nur darauf an, dass die Anwesenheit oder Abwesenheit eines Analyten richtig festgestellt werden kann, nicht jedoch in welcher Konzentration er im Blut eines Patienten vorliegt. Das ist z.B. der Fall, wenn die Blutprobe darauf untersucht wird, ob der Patient an einer Stoffwechselkrankheit leidet, die mit einem defekten Gen assoziiert ist. Die Blutprobe muss dann lediglich darauf untersucht werden, ob ein solches Gen *vorhanden* ist, nicht jedoch wie viel von dem genetischen Material umfassend das Gen in der Probe enthalten ist.

Im Falle anderer Analyten muss festgestellt werden, in welcher Konzentration der Analyt im Blut auftritt, weil ein diagnostischer Referenz-Konzentrationsbereich bekannt ist. Liegt die Konzentration des Analyten in diesem Bereich, so spricht dies dafür, dass der Patient gesund ist. Für solche semi-quantitativen oder auch rein quantitativen Bestimmungen kommt es daher darauf an, dass die Menge oder Konzentration des Analyten richtig bestimmt wird.

Für die Messung von Konzentrationen bestimmter Analyten im Blut von Patienten kann dem Patienten prinzipiell venöses Blut abgenommen, vorzugsweise gefolgt von der Aufbereitung des Blutes zu Serum. Abgesehen davon, dass dieser invasive Eingriff für den Patienten unangenehm ist und in geringem Maße sogar gesundheitliche Risiken birgt, wie das der Übelkeit oder der Ohnmacht, kann die Blutabnahme nur durch eine qualifizierte Fachkraft wie einen Arzt oder wenigstens eine Krankenschwester oder erfahrene Laborantin durchgeführt werden. Der Patient muss dazu eine Arztpraxis oder ein Krankenhaus aufsuchen.

Viel einfacher und schonender ist hingegen die Gewinnung von Kapillarblut. Nach dem Einstechen mit einem spitzen Gegenstand in die Fingerkuppe oder das Ohrläppchen des Patienten treten einige Tropfen Kapillarblut aus, die mit einer Pipette aufgenommen und auf einen absorptionsfähigen Probenträger gegeben werden. Sobald das Blut auf dem Träger eingetrocknet ist, ggf. bei Raumtemperatur vollständig nach einigen Stunden, kann der Träger ohne weitere Bearbeitung transportiert werden, sehr einfach sogar per Briefpost. Der Besuch der Arztpraxis wird damit überflüssig. Aufgrund der getrockneten Blutprobe bzw. des getrockneten Blutbestandteils gilt der Probenträger mit der darauf befindlichen Probe nicht mehr als Gefahrgut. Aus einem Träger mit eingetrocknetem Blut, bevorzugt Kapillarblut, kann ein Stück Träger mit Blut abgelöst werden. Das kann beispielsweise mit einer Vorrichtung, wie sie in der US14/900,360 beschrieben ist, oder durch Ausstanzen erreicht werden. Das abgelöste Stück Träger mit eingetrocknetem Blut wird häufig als "Dried Blood Spot" oder "DBS" bezeichnet.

Ein hier genannter Träger kann beispielsweise durch eine Membranschicht oder Membran gegeben sein, welche das Blut bzw. Blutbestandteile in einem flüssigen Aggregatzustand aufnehmen kann und auf dem dann eben das Blut bzw. Blutbestandteile eintrocknen bevor der Träger in Form der absorptionsfähigen Membran transportiert wird.

Bei dem von der Membran aufgenommenen Blutbestandteil kann es sich um Vollblut handeln, insbesondere Kapillarblut.

Um bei gleichartigen bzw. gleichen Patientenproben gleichartige bzw. vergleichbare Prozessierungsergebnisse im Zuge des Eluierens bzw. Lösens des getrockneten Blutbestandteils aus dem Membranelement zu erreichen, sodass eine Reproduzierbarkeit der Ergebnisse gewährleistet ist, ist es unter anderem notwendig, dass das Membranelement nach Einbringen in die Vertiefung beispielsweise nicht auf der Oberfläche der Flüssigkeit aufschwimmt und dadurch möglicherweise mit seiner nach oben hin gerichteten Oberseite nur ungenügend mit der flüssigen Lösung in Kontakt kommt.

Aus der WO2019/089757A1 ist ein sogenannter *Multi-Well-Plate-Adapter* bekannt, mittels welchem mehrere Rückhalteelemente bzw. Eintauchelemente gleichzeitig in korrespondierende Vertiefungen einer Multi-Well-Platte eingebracht werden können. Mittels solcher Rückhalteelemente bzw. Eintauchelemente können entsprechende Membranelemente in die jeweilige flüssige Lösung einer jeweiligen Vertiefung zum Eintauchen gezwungen werden.

Das Dokument US2007082390A1 behauptet eine Vorrichtung geeignet zum Niederhalten von Membranelementen, wobei die Vorrichtung aus einer Trägerstruktur in der Rückhalteelemente befestigt sind.

Die erfindungsgemäße Aufgabe ist es, eine einfach zu handhabende Vorrichtung bereitzustellen, mittels welcher Membranelemente in jeweiligen Vertiefungen mit jeweiligen Lösungen in einer Multi-Well-Platte sicher positioniert werden können, so dass eine Aspiration der Lösung mittels Pipettiernadeln ohne Verstopfen der Nadelöffnungen durch die Membranelemente durchgeführt werden kann.

Erfindungsgemäß wird daher eine Vorrichtung zum Niederhalten von jeweiligen Membranelementen in jeweiligen Vertiefungen einer Multi-Well-Platte vorgeschlagen. Die Multi-Well-Platte weist an ihrer Oberseite jeweilige Öffnungen der jeweiligen Vertiefungen der Multi-well-Platte auf. Die jeweiligen Vertiefungen sind jeweils zur Aufnahme einer jeweiligen flüssigen Lösung und zur Aufnahme eines jeweiligen Membranelementes geeignet.

Die Vorrichtung weist ferner an ihrem oberen Bereich eine Trägerstruktur mit jeweiligen Öffnungen der Trägerstruktur auf sowie jeweilige korrespondierende und sich von den jeweiligen Öffnungen der Trägerstruktur her nach unten hin erstreckenden Rückhalteelementen, sodass bei Auflegen der Trägerstruktur auf die Oberseite der Multi-Well-Platte jeweilige der Rückhalteelemente in jeweilige der Vertiefungen der Multi-Well-Platte hineinragen.

Erfindungsgemäß weist ein jeweiliges der Rückhalteelemente mehrere sich von der Trägerstruktur her nach unten hin erstreckenden Stege auf, deren Enden an einer Unterseite der Vorrichtung mittels eines Verbindungselementes miteinander verbunden sind, wobei das Verbindungselement eine mittlere Öffnung aufweist. Das Verbindungselement ist vorzugsweise ringförmig.

Ferner weist erfindungsgemäß ein jeweiliges der Rückhalteelemente zwischen den Stegen mehrere Öffnungen auf bzw. zwischen den Stegen werden mehrere Öffnungen gebildet. Diese Öffnungen zwischen den Stegen erstrecken sich von der Trägerstruktur her bis hin zu dem Verbindungselement durchgehend. Ferner erstrecken sich diese Öffnungen bei Auflegen der Trägerstruktur auf die Oberseite der Multi-Well-Platte von der Oberseite der Multi-Well-Platte her bzw. von der Öffnung der korrespondierenden Vertiefung der Multi-Well-Platte her durchgehend bis hin zu dem Verbindungselement. Das Verbindungselement befindet sich insbesondere an der Unterseite der Vorrichtung.

Zur Darlegung eines oder mehrerer möglicher Vorteile der Erfindung erfolgen nun genauere Ausführungen.

Für eine vorzugsweise automatisierte Abarbeitung eines biochemischen Verfahrens, in dessen Zuge die Patientenprobe aus dem Membranelement herausgelöst werden muss, kann es notwendig sein, nach Lösen bzw. Eluieren der Probe aus dem Membranelement zum Überführen der Probenbestandteile in die flüssige Lösung anschließend die flüssige Lösung aus der Vertiefung der Multi-Well-Platte zu aspirieren und in andere Reaktionsgefäße zu überführen. Dieses erfolgt insbesondere vorzugsweise mittels einer Aspirationsnadel bzw. Pipettiernadel. Dadurch, dass die Rückhalteelemente entsprechende Membranelemente bei Eintauchen der Vorrichtung bzw. Eintauchen der Rückhalteelemente in den entsprechenden Vertiefungen der Multi-Well-Platte in einem unteren Bereich der Vertiefung sicher positionieren bzw. festhalten, können Aspirationsnadeln in die jeweilige Vertiefungen der Multi-Well-Platte eingebracht bzw. abgesenkt werden und dann eine Aspiration der flüssigen Lösung vorgenommen werden, ohne dass ein Verstopfen der Nadeln durch ein Aufschwimmen der Membranelemente hin zu den Öffnungen der Nadeln befürchtet werden muss. Es verhindert also die erfindungsgemäße Vorrichtung beim Transfer der eluierten Proben aus der Multi-Well-Platte in andere Reaktionsgefäße, dass die im Eluat schwimmenden Membranelemente unbemerkt zu einem Verschluss der Pipettiernadel führen. Hierdurch wird somit ein korrekter Transfer eines bestimmten Volumens der Flüssigkeit mittels der Pipettiernadel sichergestellt.

Die erfindungsgemäße Vorrichtung kann also so verwendet werden, dass die jeweiligen Rückhalteelemente in jeweilige Vertiefungen der Multi-Well-Platte bzw. Elutionsplatte eingetaucht werden, wobei hierbei darauf zu achten ist, dass keine flüssige Lösung bzw. kein Eluat über den Rand eines bestimmten Wells bzw. einer bestimmten Vertiefung austritt.

Im Zuge einer Prozessierung mehrerer Patientenproben bzw. mehrerer Membranelemente in jeweiligen Vertiefungen der Multi-Well-Platte stellt jede Vertiefung eine eigene, jeweilige Prozessierung einer eigenen, jeweiligen Probe dar. Ein Übertreten von flüssiger Lösung aus einer Vertiefung in eine andere Vertiefung würde die entsprechenden Prozessierungsergebnisse verfälschen und falsche Resultate hervorrufen. Dadurch, dass erfindungsgemäß zwischen den Stegen jeweilige Öffnungen vorhanden sind, kann ein jeweiliges Rückhalteelement so in die Flüssigkeit einer Vertiefung eingetaucht werden, dass die flüssige Lösung diese Öffnungen durchfließen kann, sodass eine Volumenverdrängung der flüssigen Lösung durch die Rückhalteelemente minimiert wird und somit das Risiko eines Aufsteigens der Flüssigkeit aus der Vertiefung heraus bis hin zur Oberseite der Multi-Well-Platte minimiert wird. Hierdurch wird das Risiko eines Übertretens von flüssiger Lösung aus einer Vertiefung heraus hin zu einer anderen Vertiefung minimiert, so dass eine Kreuzkontamination zwischen den jeweiligen Proben der jeweiligen Vertiefungen vermieden wird.

Insbesondere dadurch, dass das Verbindungselement der Stege im unteren Bereich der Vorrichtung eine mittlere Öffnung aufweist, unterstützt diese mittlere Öffnung abermals das Minimieren einer Volumenverdrängung der Flüssigkeit durch das Rückhalteelement, da Flüssigkeit auch durch diese mittlere Öffnung während des Einbringens der Rückhalteelemente in die Vertiefungen bzw. die Flüssigkeiten hindurchströmen kann. Es wird also auch hierdurch verhindert, dass bei Eintauchen des Rückhalteelementes in die Vertiefung die Flüssigkeit einer ersten Probe bis zur Oberseite der Multi-Well-Platte aufsteigt und möglicherweise von einer Vertiefung in eine andere Vertiefung mit einer anderen Flüssigkeit einer anderen Probe überführt wird. Ferner wird durch die mittlere Öffnung des Verbindungselementes verhindert, dass das Membranelement bei Eintauchen des Rückhalteelementes seitlich wegschwimmt, da das Membranelement mittig in der Vertiefung positioniert bleibt.

Ferner wird durch die Verwendung der erfindungsgemäßen Vorrichtung erreicht, dass die Membranelemente immer in gleichartiger Weise in die flüssige Lösung eingetaucht werden, sodass Prozessierungsergebnisse reproduzierbar werden.

Vorteilhafte Ausführungsformen der Erfindung sind Gegenstand der abhängigen Patentansprüche und werden in der folgenden Beschreibung unter teilweise Bezugnahme auf die Figuren näher erläutert.

Vorzugsweise bilden jeweilige Unterseiten der Verbindungselemente die Unterseite der Vorrichtung. Hierbei liegt vorzugsweise die Unterseite der Vorrichtung in einer Ebene bzw. bildet eine Ebene, welche parallel zu einer Ebene der Trägerstruktur ist.

Hierdurch wird eine definierte Tauchtiefe der Membranelemente gegenüber der Ebene der Trägerstruktur und somit auch gegenüber der Oberseite der Multi-Well-Platte klar definiert und herbeigeführt.

Vorzugsweise ist ein jeweiliges der Rückhalteelemente so ausgebildet, dass entlang einer mittleren Symmetrieachse des Rückhalteelementes von oben her durch die jeweils korrespondierende Öffnung an der Trägerstruktur eine Pipettiernadel nach unten hin bis hin zu dem insbesondere ringartigen Verbindungselement eingeführt werden kann. Hierdurch wird es ermöglicht, dass die flüssige Lösung mittels einer Pipettiernadel überführt werden kann, wobei aufgrund des Niederhaltens bzw. Eintauchens des Membranelementes durch das Rückhalteelement der Pipettiernadel ein bestimmter Eintauchraum bzw. eine bestimmte Eintauchtiefe ermöglicht wird.

Erfindungsgemäß sind die Stege symmetrisch, insbesondere rotationssymmetrisch, um eine mittlere Symmetrieachse des Rückhalteelementes angeordnet. Hierbei laufen die Stege von oben her bzw. von der Trägerstruktur her nach unten hin insbesondere hin zu den Verbindungselementen bzw. hin zur Unterseite der Vorrichtung konisch aufeinander zu. Hierdurch wird es in vorteilhafterweise ermöglicht, dass es genügend Platz zwischen den Stegen und einer möglicherweise konisch verlaufenden Innenseite einer Vertiefung gibt, sodass ein Aufsteigen der Lösung bis hin zu der Oberseite der Multi-Well-Platte bzw. bin hin zu einer Öffnung einer Vertiefung vermieden bzw. minimiert wird.

Vorzugsweise weist die Vorrichtung an jeweils gegenüberliegenden Enden der Trägerstruktur jeweilige Halterungen auf, welche durch einen Nutzer gegriffen werden können. Diese Halterungen erstrecken sich vorzugsweise entlang einer Ebene der Trägerstruktur. Hierdurch kann ein Nutzer in vorteilhafter Weise die Vorrichtung greifen und dann das Eintauchen der jeweiligen Rückhalteelemente in die jeweiligen Vertiefungen herbeiführen, bis die Trägerstruktur auf der Oberseite der Multi-Well-Platte aufliegt.

Vorzugsweise weist jede der Halterungen an ihrer jeweiligen Unterseite wenigstens ein Beabstandungselement auf, sodass bei Auflegen der Vorrichtung bzw. der Trägerstruktur auf die Oberseite der Multi-Well-Platte die Beabstandungselemente ein Beabstanden einer Unterseite der Trägerstruktur von der Oberseite der Multi-Well-Platte bewirken. Hierdurch wird in vorteilhafterweise vermieden, dass die Unterseite der Trägerstruktur direkt auf der Oberseite der Multi-Well-Platte aufliegt. Wäre dies der Fall, so könnte Flüssigkeit, welche aus einer Vertiefung heraus bis zur Unterseite der Trägerstruktur her aufsteigt, aufgrund eines Kapillareffektes zwischen der Unterseite der Trägerstruktur und der Oberseite der Multi-Well-Platte von einer Vertiefung her zu einer anderen Vertiefung hin transportiert bzw. geleitet werden, welches eine Kreuzkontamination der Proben herbeiführen könnte. Dieses wird aufgrund der vorgeschlagenen Beabstandungselemente vermieden.

Vorzugsweise weist die Vorrichtung mehrere, entlang einer geraden Linie angeordnete Öffnungen und dazu korrespondierende Rückhalteelemente auf, wobei die Vorrichtung ferner zwischen zwei Öffnungen an der Unterseite der Trägerstruktur ein weiteres Beabstandungselement aufweist, welches bei Auflegen der Trägerstruktur auf die Oberseite der Multi-Well-Platte ein Beabstanden der Unterseite der Trägerstruktur von der Oberseite der Multi-Well-Platte bewirkt. Diese Ausführungsform ist vorteilhaft, da in dem Fall, dass die Vorrichtung mehrere, hinterinander angeordnete Rückhalteelemente aufweist, dann auch hier in einem mittleren Bereich, nämlich an dem Ort des weiteren Beabstandungselementes, die Unterseite der Vorrichtung von der Oberseite der Multi-Well-Platte so beabstandet wird, dass aufgrund von Kapillarkräften keine Flüssigkeit von einer Vertiefung in eine andere eintritt. Vorzugsweise führt das weitere Beabstandungselement und/oder die zuvor genannten Beabstandungselemente ein Abstand von 2 mm zwischen der Oberseite der Platte bzw. der Multi-Well-Platte und der Unterseite der Trägerstruktur herbei.

Erfindungsgemäß sind die Stege eines Rückhalteelementes auf einem äußeren Rand der jeweiligen Öffnung der Trägerstruktur rotationssymmetrisch um die mittlere Symmetrieachse des Rückhalteelementes angeordnet. Hierbei sind Erfindungsgemäß an ersten Randabschnitten dieses Randes die Stege angeordnet und ferner sind an zweiten Randabschnitten des Randes die sich nach unten hin erstreckenden Öffnungen zwischen den Stegen angeordnet. Ferner sind Erfindungsgemäß zueinander nächstliegende, benachbarte bzw. unmittelbar benachbarte Randabschnitte zueinander benachbarter Öffnungen der Trägerstruktur jeweils Randabschnitte der zweiten Art bzw. zweite Randabschnitte. Hierdurch wird in vorteilhafterweise erreicht, dass ein Übertreten von Flüssigkeit aus einer Vertiefung in eine andere Vertiefung ferner minimiert wird, da Flüssigkeit, welche aufgrund von Kapillarkräften zwischen einem Steg und einer Innenseite einer Vertiefung hoch bis zu der Oberseite der Multi-Well-Platte tritt, dann keinen kürzesten Weg von dieser einen Vertiefung zu einer nächsten Vertiefung finden kann; ein solcher kürzester Weg zwischen zwei benachbarten Vertiefungen ist nämlich der Weg zwischen zwei am nächsten benachbarten Randabschnitten der zweiten Art.

Vorgeschlagen wird ferner ein Kit aufweisend eine Multi-Well-Platte und eine Vorrichtung der erfindungsgemäßen Art.

Vorzugsweise verbleibt bei dem Kit bei Einsetzen der Vorrichtung in die Multi-Well-Platte zwischen einer Unterseite der Vorrichtung bzw. einer Unterseite des Verbindungselementes und einer Bodenseite einer Vertiefung eine Lücke von wenigstens 1 mm.

Vorzugsweise verbleibt bei Einsetzen der Vorrichtung in die Multi-Well-Platte zwischen einem Steg und einer Innenseite einer Vertiefung in insbesondere einem seitlichen Randbereich der Vertiefung eine Lücke von maximal 0,9 mm, bevorzugt 0,8 mm, besonders bevorzugt 0,7 mm, ganz besonders bevorzugt 0,6 mm.

Diese Dimensionierung der Lücke zwischen dem Steg und der Innenseite der Vertiefung hat den Vorteil, dass das Membranelement sich nicht in diesem seitlichen Bereich bewegen kann, da es eine Mindestdicke aufweist, sodass das Membranelement nicht aufschwimmen kann, aber trotzdem frei unterhalb des Verbindungselementes der Stege schwimmen kann und nicht eingeklemmt wird.

Vorgeschlagen wird ferner ein Verfahren zum Nachweis eines Analyten in getrockneten Blutbestandteilen umfassend die Schritte Bereitstellen eines Membranelementes, welches die getrockneten Blutbestandteile aufweist, Einbringen des Membranelementes in eine Vertiefung einer Multi-Well-Platte, Einbringen einer Flüssigkeit, die geeignet ist, um den Analyten aus der eingetrockneten Blutprobe aufzunehmen, in die Vertiefung, Ineinanderfügen der Multi-Well-Platte mit einer erfindungsgemäßen Vorrichtung, so dass das Membranelement durch die Vorrichtung in einem unteren Bereich der Vertiefung unterhalb der Flüssigkeitsoberfläche positioniert wird, Einführen einer Pipettiernadel in die Vertiefung und Aspirieren zumindest eines Teilvolumens der Flüssigkeit, Überführen des Teilvolumens der Flüssigkeit in ein Aufnahmegefäß bzw. Reaktionsgefäß sowie Nachweisen des Analyten in dem Teilvolumen der Flüssigkeit.

Unter dem Begriff "quantitative Bestimmung" wird im Sinne dieser Anmeldung eine Bestimmung verstanden, die eine Aussage über die absolute Konzentration des Analyten gestattet, noch bevorzugter mit einem numerischen Wert. Es kann sich alternativ um eine semiquantitative Bestimmung handeln, bei der eine Zuordnung der Konzentration zu einem Bereich aus wenigstens drei, möglichst vier Konzentrationsbereichen ermöglicht wird, z.B. negativ, schwach positiv und positiv, oder eine relative Konzentrationsbestimmung. Insbesondere erfolgt die Konzentrationsbestimmung mit Hilfe von Kalibratoren, wobei es sich bevorzugt um zwei oder mehr, bevorzugt vier Einheiten, bevorzugt Lösungen oder auf einem diagnostisch nützlichen Träger gecoateten festen Analyten handelt, die jeweils eine bekannte Menge des Analyten enthalten, wobei die zwei oder mehr Einheiten jeweils eine andere bekannte Menge umfassen.

Eine quantitative Bestimmung wird vorzugsweise mit einem Verfahren ausgeführt, das aus der Gruppe ausgewählt ist, die Immundiffusion, Immunelektrophorese, Lichtstreuung, Agglutination und Immuntest mit Markierung - wie die aus der Gruppe umfassend Immuntest mit radioaktiver Markierung, mit enzymatischer Markierung, bevorzugter ELISA, mit Chemilumineszenz-Markierung, bevorzugter Elektrochemilumineszenz-Markierung und mit Immunfluoreszenz-Markierung, bevorzugter indirekte Immunfluoreszenz-Markierung - umfasst, bevorzugt mit ELISA.

Der getrocknete Blutbestandteil wird aus dem Membranelement durch Kontaktieren des Membranelementes mit der Flüssigkeit eluiert, die geeignet ist, um den Analyten aus dem eingetrockneten Blutbestandteil aufzunehmen. Dabei kommen insbesondere wässrige Puffer mit geeignetem pH und Salzgehalt in Frage, beispielsweise PBS. Die genaue Zusammensetzung der Flüssigkeit und die Bedingungen und die Dauer des Kontaktierens können durch Routineversuche zur Stabilisierung und Optimierung zwecks möglichst vollständiger Aufnahme des Analyten in die Flüssigkeit herausgefunden werden und hängen von der Natur des Analyten ab. Die Flüssigkeit wird möglichst auch gleich derart gewählt, dass sie mit dem nachfolgend eingesetzten Verfahren zum Nachweisen des Analyten kompatibel ist. Anschließend wird der Analyt von Interesse in der Flüssigkeit nachgewiesen. Dabei wird bestimmt, ob der Analyt anwesend oder abwesend bzw. in einer Konzentration oberhalb der Nachweisgrenze der eingesetzten Nachweismethode vorhanden ist. Bevorzugt wird der Analyt semi-quantitativ oder quantitativ nachgewiesen. Verschiedene Optionen zur Durchführung des Verfahrens sind im Stand der Technik beschrieben, z.B. Grüner, N., Stambouli, O. und Ross, R. S. (2015) Dried Blood Spots - Preparing and Processing for Use in Immunoassays and in Molecular Techniques, J. Vis. Exp 97, 52619.

In einer bevorzugten Ausführungsform wird unter dem Begriff "Analyt", wie hierin verwendet, ein in der Blutprobe vorhandener Stoff verstanden, der beim Eintrocknen der Blutprobe auf dem Aufnahmebereich des Trägers verbleibt und von dort für die quantitative Bestimmung in eine andere Flüssigkeit überführt werden kann. Besonders bevorzugt sind in wässrigen Lösungen gut lösliche Stoffe, die in der Blutprobe aufgelöst waren. Möglich sind jedoch auch Stoffe wie feste Partikel, die in der Lösung in Form einer Suspension vorliegen. Auch ihre Konzentration in der Blutprobe oder einer wässrigen Lösung kann mit geeigneten physikalischen Messverfahren wie dem Nachweis von Lichtstreuung quantitativ bestimmt werden. In einer besonders bevorzugten Ausführungsform ist der Analyt aus der Gruppe ausgewählt, die einen Metaboliten, ein Protein, eine Nukleinsäure und ein Lipid umfasst und ist besonders bevorzugt ein Antikörper, noch bevorzugter ausgewählt aus der Gruppe von Klassen, die IgA, IgM, IgG und IgE umfassen, am bevorzugtesten IgG. In einer bevorzugten Ausführungsform handelt es sich bei dem Antikörper um einen Antikörper von einem Säugetier, noch bevorzugter von einem Menschen.

In einer bevorzugten Ausführungsform wird unter dem Begriff "absorptionsfähig", wie hierin verwendet, verstanden, dass das so bezeichnete Material in der Lage ist, einen Bluttropfen aufzunehmen, wobei der Wasseranteil zunächst aufgenommen und anschließend beim Trocknen an die Umgebung abgegeben wird, wobei die darin gelösten Komponenten, darunter der Analyt wie ein Antikörper, im Material verbleiben, und durch erneutes Kontaktieren mit einem geeigneten Lösungsmittel, bevorzugt einem wässrigen Puffer, wie herausgelöst werden können. In einer bevorzugten Ausführungsform besteht der Aufnahmebereich aus nicht gewobenem Polyolefin, abgesehen davon, dass handelsübliche Verunreinigungen oder Zusatzstoffe anwesend sein können.

Im Folgenden wird die Erfindung anhand spezieller Ausführungsformen ohne Beschränkung des allgemeinen Erfindungsgedankens anhand der Figuren näher erläutert. Dabei zeigen:
Fig. 1 eine bevorzugte Ausführungsform der erfindungsgemäßen Vorrichtung aus einer ersten Perspektive,
Fig. 2 die Ausführungsform der Vorrichtung aus einer zweiten Perspektive,
Fig. 3 eine Seitenansicht der Ausführungsform der Vorrichtung,
Fig. 4 eine Ansicht von unten der Ausführungsform der Vorrichtung,
Fig. 5 eine Aufsicht von oben der Ausführungsform der Vorrichtung,
Fig. 6 die Ausführungsform der Vorrichtung zusammen mit einer Multi-Well-Platte bei Einsetzen der Vorrichtung in die Multi-Well-Platte,
Fig. 7 eine Schnittansicht der Ausführungsform der Vorrichtung zusammen mit der Multi-Well-Platte,
Fig. 8 eine Schrägansicht von unten der Ausführungsform der Vorrichtung,
Fig. 9 eine weitere Schrägansicht der Ausführungsform der Vorrichtung,
Fig. 10 eine Schnittansicht einer Vertiefung einer Multi-Well-Platte zusammen mit einer Schnittansicht eines unteren Bereiches der Ausführungform der Vorrichtung,
Fig. 11a, b, c in Vertiefungen eingesetzte Rückhalteelemente der Ausführungsform der Vorrichtung zusammen mit verschiedenen Pegelständen einer flüssigen Lösung sowie
Fig. 12 bis 15 Experimentalergebnisse.

Verschiedene Ausführungsbeispiele werden nun ausführlicher unter Bezugnahme auf die beiliegenden Zeichnungen beschrieben, in denen einige Ausführungsbeispiele dargestellt sind.

Bei der nachfolgenden Beschreibung der beigefügten Figuren, die lediglich einige exemplarische Ausführungsbeispiele zeigen, können gleiche Bezugszeichen gleiche oder vergleichbare Komponenten bezeichnen. Ferner können zusammenfassende Bezugszeichen für Komponenten und Objekte verwendet werden, die mehrfach in einem Ausführungsbeispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt. Optionale Komponenten sind in den Figuren mit gestrichelten Linien oder Pfeilen dargestellt.

Obwohl Ausführungsbeispiele auf verschiedene Weise modifiziert und abgeändert werden können, sind Ausführungsbeispiele in den Figuren als Beispiele dargestellt und werden hierin ausführlich beschrieben. Es sei jedoch klargestellt, dass nicht beabsichtigt ist, Ausführungsbeispiele auf die jeweils offenbarten Formen zu beschränken, sondern dass Ausführungsbeispiele vielmehr sämtliche funktionale und/oder strukturelle Modifikationen, Äquivalente und Alternativen, die im Bereich der Erfindung liegen, abdecken sollen. Gleiche Bezugszeichen bezeichnen in der gesamten Figurenbeschreibung gleiche oder ähnliche Elemente.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

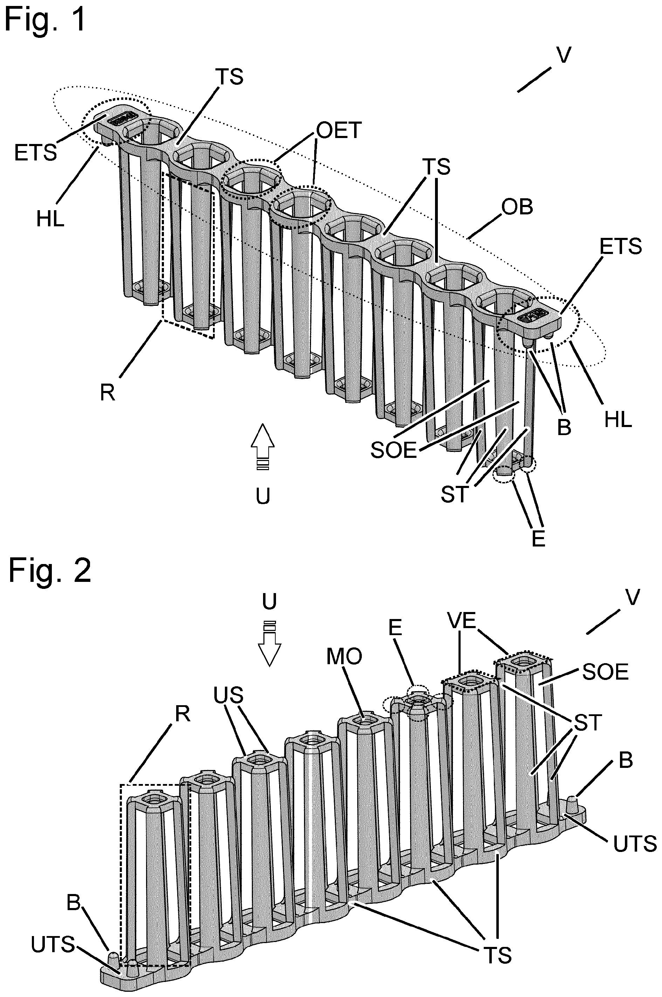

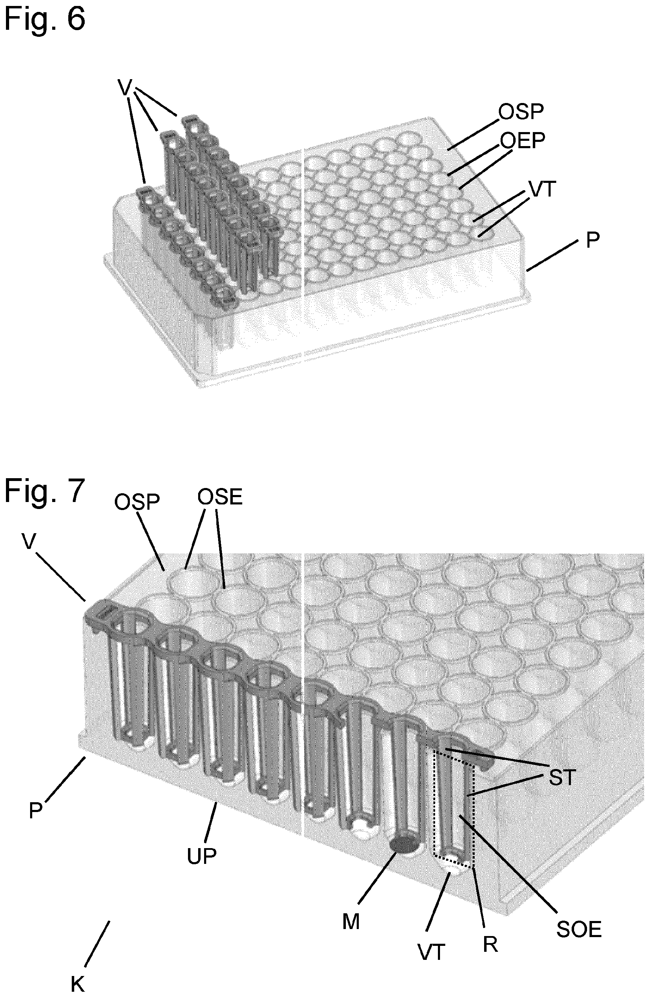

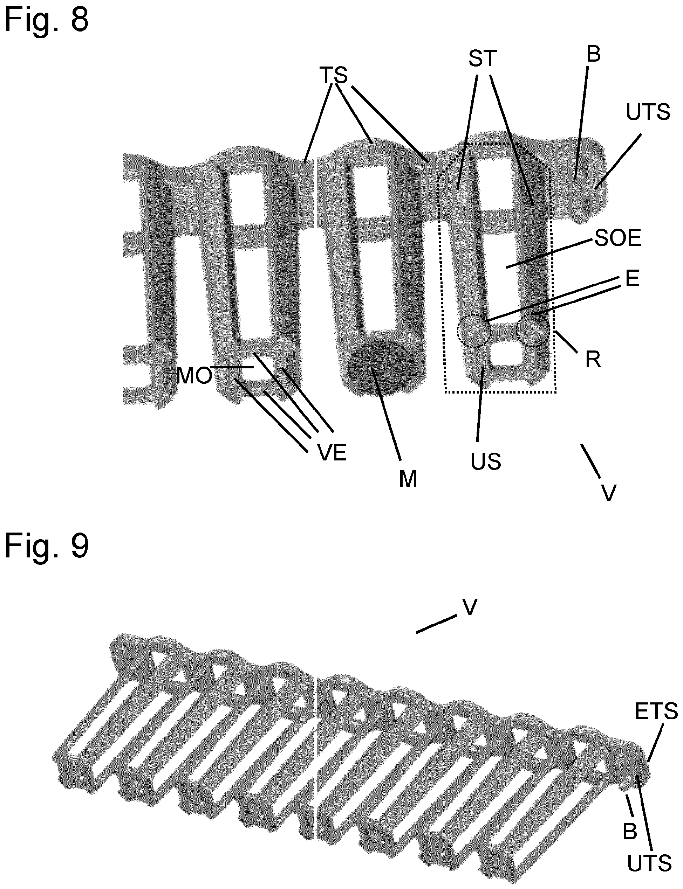

Die Fig. 1 zeigt eine Vorrichtung V zum Niederhalten von Membranelementen. Die Fig. 7 als auch die Fig. 8 zeigen ein entsprechendes Membranelement M.

Fig. 6 zeigt eine Multi-Well-Platte P zusammen mit der Vorrichtung V, welche in die Multi-Well-Platte P eingesetzt ist bzw. eingeführt wird.

Die Platte P ist eine Multi-Well-Platte P, vorzugsweise eine Mikrotiterplatte, insbesondere eine sogenannte Deep-Well-Platte. Besonders bevorzugt ist die Platte P eine SBS-Verdünnungsplatte. Die Multi-Well-Platte P ist vorzugsweise aus Plastik. Vorzugsweise weist die Multi-Well-Platte eine bestimmte Anzahl von Vertiefungen aus, wobei diese Anzahl aus der Gruppe bestehend aus sechs Vertiefungen, zwölf Vertiefungen, 24 Vertiefungen, 48 Vertiefungen, 96 Vertiefungen, 384 Vertiefungen, 1536 Vertiefungen und 3456 Vertiefungen ausgewählt ist. Ganz besonders bevorzugt ist die Multi-Well-Platte von der Art, dass sie 96 Vertiefungen aufweist.

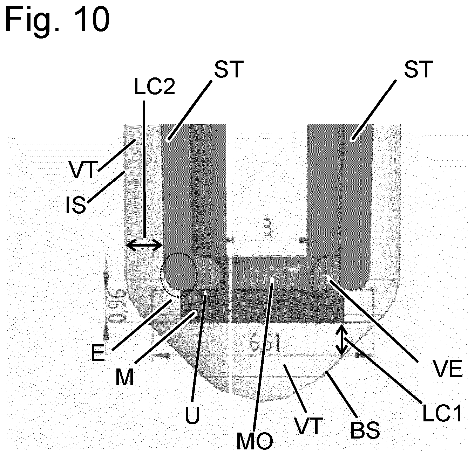

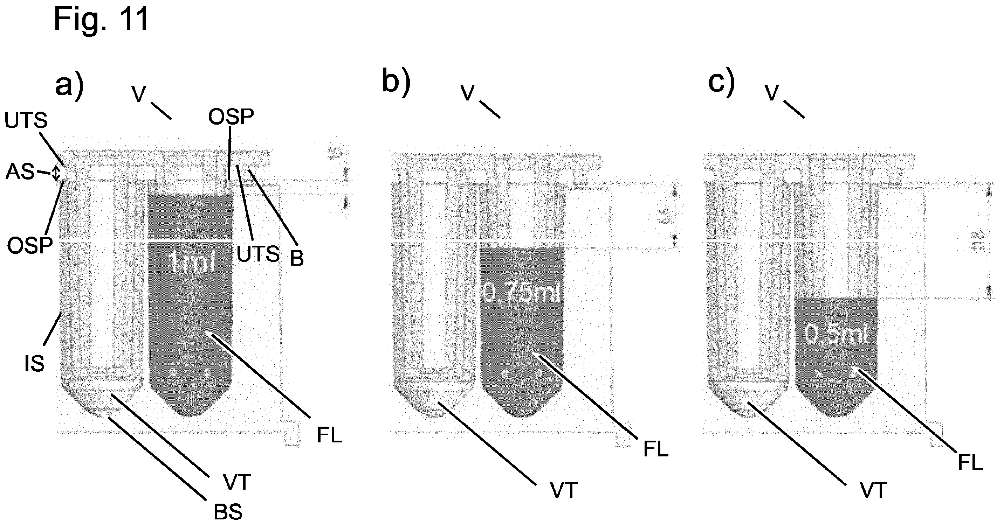

Die Fig. 7 zeigt hierzu auch noch einmal die Multi-Well-Platte P, welche an ihrer Oberseite OSP jeweilige Öffnungen OEP jeweiliger Vertiefungen VT aufweist. Solche Vertiefungen VT sind auch in der Fig. 6 ersichtlich. Solche Vertiefungen VT sind auch in den Fig. 11a, b sowie c dargestellt, aus welchen ersichtlich wird, dass die jeweiligen Vertiefungen VT jeweils zur Aufnahme einer flüssigen Lösung FL geeignet sind. Die Vertiefungen VT sind an einer Unterseite UP der Platte P nach unten hin abgeschlossen bzw. verschlossen und weisen einen Boden bzw. eine Bodenseite BS auf, wie in der Fig. 10 dargestellt.

Die Fig. 10 zeigt hierzu eine Vergrößerung eines Teilbereiches einer Vertiefung VT gemeinsam mit einer Schnittansicht eines unteren Bereiches einer erfindungsgemäßen Vorrichtung, wobei aus der Fig. 10 auch heraus hervorgeht, dass eine Vertiefung VT zur Aufnahme eines Membranelementes M ausgebildet ist.

In der Fig. 1 ist klar dargestellt, dass die Vorrichtung V an ihrem oberen Bereich OB - indiziert mittels einer gestrichelten Ellipsenkurve, die nicht Teil der Vorrichtung ist - eine Trägerstruktur TS mit jeweiligen Öffnungen OET aufweist, welche jeweils mittels gestrichelter Kreislinien indiziert sind. Ferner weist die Vorrichtung V jeweilige zu den jeweiligen Öffnungen OET korrespondierende sich nach unten hin erstreckende Rückhalteelemente R auf.

Wie aus der Fig. 6 und der Fig. 7 klar hervorgeht, ist die Vorrichtung V so ausgestaltet, dass jeweilige Rückhalteelemente R in jeweilige Vertiefungen VT der Multi-Well-Platte eingebracht werden können, sodass bei Auflegen der Trägerstruktur TS auf die Oberseite OSP der Multi-Well-Platte P die jeweiligen Rückhalteelemente R in die jeweiligen Vertiefungen VT hineinragen.

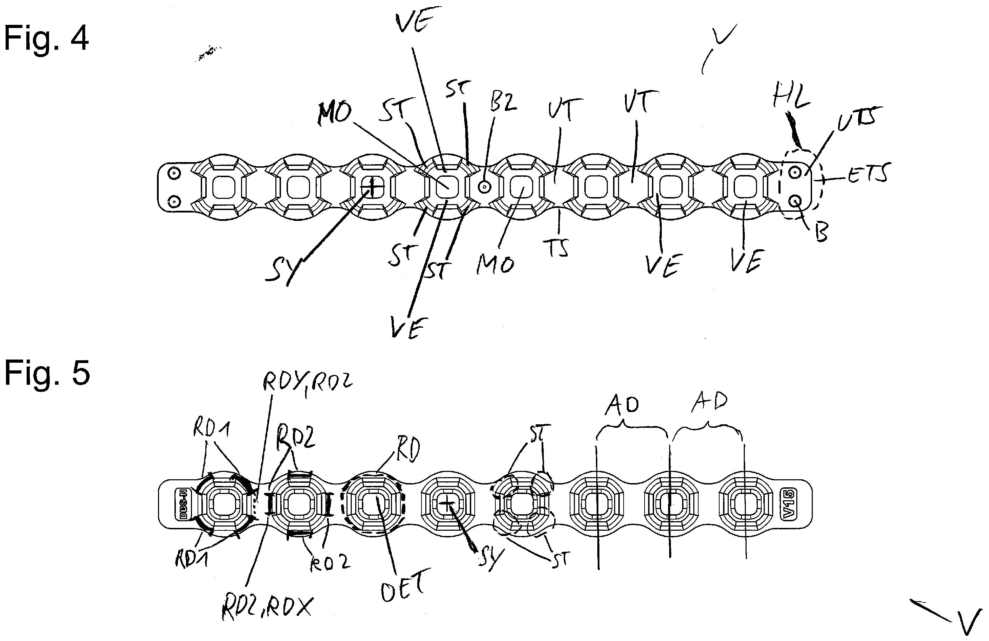

Wie aus der Fig. 1 ersichtlich, weist ein jeweiliges der Rückhalteelemente R mehrere sich von der Trägerstruktur TS her nach unten hin erstreckende Stege ST auf. Die Enden E dieser Stege ST sind an einer Unterseite U der Vorrichtung V mittels eines Verbindungselementes VE, welches auch in der Fig. 10 und auch in der Fig. 4 dargestellt ist, miteinander verbunden. Wie aus der Fig. 4 ebenfalls hervorgeht, weist ein solches insbesondere ringartiges Verbindungselement VE eine mittlere Öffnung MO auf.

Auch die Fig. 8 zeigt hierzu aus einer Schrägansicht von unten ein entsprechendes Verbindungselement VE mit einer mittleren Öffnung MO.

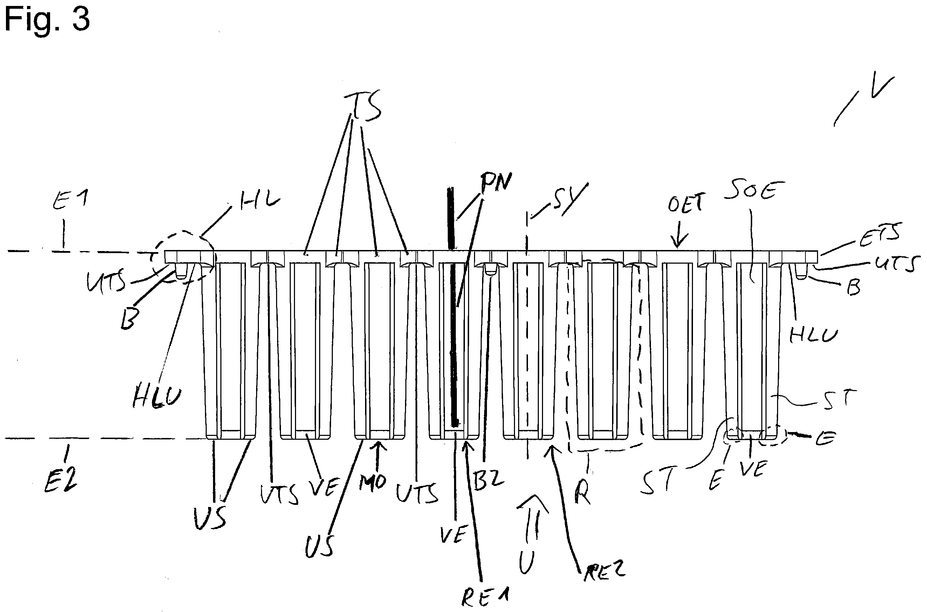

Aus der Fig. 1 geht ebenfalls hervor, dass ein jeweiliges der Rückhalteelemente R zwischen den Stegen ST des Rückhalteelementes R jeweilige Öffnungen SOE aufweist. Eine solche Öffnung SOE zwischen zwei Stegen ST ist ebenfalls in der Fig. 3 in der Seitenansicht der Vorrichtung V eingezeichnet. Eine solche Öffnung SOE zwischen zwei Stegen ST erstreckt sich von der Trägerstruktur TS bzw. der Unterseite der Trägerstruktur UTS her bis hin zu dem Verbindungselement VE durchgehend, wobei eine Unterseite US des Verbindungselementes VE insbesondere die Unterseite U der Vorrichtung V bildet. Wie ferner aus der Fig. 7 hervorgeht, erstreckt eine solche Öffnung SOE zwischen zwei Stegen ST sich bei Auflegen der Trägerstruktur TS auf die Oberseite OSP der Multi-Well-Platte P von der Oberseite OSP der Multi-Well-Platte P her bis hin zu dem Verbindungselement VE. Hierbei sind dann insbesondere auch die Rückhalteelemente R der Vorrichtung V in die Vertiefungen VT eingeführt.

Aufgrund der hier ausgeführten Dimensionierung der Öffnungen SOE zwischen zwei Stegen ST eines Rückhalteelementes R ergibt sich eine Maximierung einer Durchflussmöglichkeit von flüssiger Lösung FL, siehe Fig. 11, bei Einbringen der Rückhalteelemente R in die Vertiefungen VT, wie in der Fig. 7 dargestellt. Hierdurch wird ein Aufsteigen von flüssiger Lösung bis hin zu der Oberseite OSP der Multi-Well-Platte P minimiert.

Die Fig. 2 zeigt für ein Rückhalteelement R als auch die Unterseite US eines Verbindungselementes VE. Eine solche Unterseite US eines Verbindungselementes VE ist abermals in der Fig. 3 eingezeichnet. Die jeweiligen Unterseiten US der Verbindungselemente VE bilden die Unterseite U der Vorrichtung V. Die Unterseite U der Vorrichtung V liegt in einer Ebene E2 bzw. bildet eine Ebene E2, welche parallel zu einer Ebene E1 der Trägerstruktur TS ist.

Werden die Rückhalteelemente R in entsprechenden Vertiefungen VT der Multi-Well-Platte P eingebracht, so wird in einer jeweiligen Vertiefung VT ein in der Vertiefung VT befindliches Membranelement M mittels der Unterseite US eines Verbindungselementes VE bzw. der Unterseite U der Vorrichtung V in einem unteren Bereich der Vertiefung VT gehalten bzw. positioniert. Vorzugsweise erfolgt dies für mehrere Membranelemente M in einer einzelnen Vertiefung VT.

Die Fig. 10 zeigt hierzu eine Schnittansicht einer Vertiefung VT einer Multi-Well-Platte bei einem in der Vertiefung VT befindlichen Rückhalteelement R. Auch hier wird ersichtlich, dass die Stege ST durch das Verbindungselement VE zusammengehalten werden an ihren Enden E, indiziert durch eine gestrichelte Ellipsenkurve. Die Unterseite U der Vorrichtung V positioniert das Membranelement M innerhalb der Vertiefung VT.

Durch Positionierung des Membranelementes M in der Weise, welche in der Fig. 10 dargestellt ist, wird es ermöglicht, dass eine Pipettiernadel von oben her in die Öffnung OET der Trägerstruktur und die Öffnung OEP der Vertiefung VT eingeführt bzw. eingebracht werden kann bis zu einer bevorzugten Tiefe, welche gerade etwas geringer als die Tiefe des Verbindungselementes VE ist, sodass auch dann noch ein Verstopfen einer Öffnung der Pipettiernadel durch das Membranelement M verhindert wird.

Das Membranelement M kann sich immer noch in dem unteren Bereich der Vertiefung VT bewegen und wird auch an seiner Oberseite hinreichend durch Flüssigkeit berührt bzw. durchtränkt.

Ferner wird insbesondere durch die durchgehende Erstreckung der Öffnungen SOE zwischen den Stegen ST und die mittlere Öffnung MO des Verbindungselementes VE erreicht, dass eine Volumenverdrängung der Flüssigkeit FL durch die Vorrichtung V minimiert wird. Würden sich lediglich in einem unteren Bereich eines Rückhalteelementes R eine oder mehrere Öffnungen befinden um ein Durchströmen der Vorrichtung durch eine Flüssigkeit zu ermöglichen, so würde bei Eintauchen der Rückhalteelemente in die Vertiefungen möglicherweise eine zu starke Volumenverdrängung der Flüssigkeit FL herbeigeführt werden, welches ein Aufsteigen der Flüssigkeit FL bis hin zu der Oberseite OSP der Multi-Well-Platte OE und so eine Kreuzkontamination zwischen den verschiedenen Proben verschiedener Vertiefungen verursachen könnte.

Die Fig. 3 zeigt ein Beispiel, bei welchem eine Pipettiernadel PN entlang einer Symmetrieachse SY des Rückhalteelementes R von oben her durch die Öffnung OET der Trägerstruktur TS bis hin nach unten hin zu dem Verbindungselement VE eingeführt ist.

Wie aus der Fig. 11a ersichtlich wird, weisen vorzugsweise die Vertiefungen VT von der Oberseite OSP der Platte P nach unten hin zur Bodenseite BS einen konischen Verlauf auf.

Wie aus der Fig. 1 als auch der Fig. 5 hervorgeht, sind die Stege ST eines Rückhalteelementes R an einem Randbereich RD einer Öffnung OET der Trägerstruktur TS befestigt bzw. erstrecken sich von diesem Randbereich RD einer Öffnungen OET her nach unten hin. Hierdurch wird der gesamte Öffnungsbereich OET zum Einbringen der Pipettiernadel PN aus der Fig. 3 bereitgestellt, sodass eine höhere Toleranz bezüglich einer Varianz der Nadelposition bzw. Positionierung der Nadel PN in einer xy-Ebene, in welcher die Trägerstruktur verläuft bzw. entlang welcher die Trägerstruktur verläuft, erreicht wird. Leichte Ausrichtungsvarianzen der Position der Pipettiernadel PN in dieser xy-Ebene bzw. der Ebene der Trägerstruktur bzw. der Ebene der Öffnung OET werden hierdurch tolerabel.

Wie aus der Fig. 5 ferner hervorgeht, sind die Stege ST symmetrisch, insbesondere rotationssymmetrisch, um eine mittlere Symmetrieachse SY des Rückhalteelementes R angeordnet. Die Symmetrieachse Y steht insbesondere senkrecht zur Ebene E1 der Trägerstruktur TS. Wie aus der Fig. 3 als auch der Fig. 7 klar hervorgeht, laufen die Stege ST von der Trägerstruktur TS von oben her nach unten hin zur Unterseite U der Vorrichtung V hin konisch aufeinander zu. Die Fig. 11a illustriert, dass hierdurch bei konisch verlaufenden Vertiefungen VT genügend Platz zwischen den Stegen ST und der Innenseite IS einer Vertiefung gegeben ist. Hierdurch wird bei konisch verlaufenden Vertiefungen aufgrund des konischen Verlaufs der Stege ein Kapillareffekt zwischen den Innenseiten IS der Vertiefung VT und den Stegen ST minimiert, sodass ein Aufsteigen der Lösung aufgrund von Kapillarkräften bis hin zu den Öffnungen OEP der Multi-Well-Platte P minimiert wird.

Die Fig. 1 zeigt, wie ebenso die Fig. 4, dass die Vorrichtung V an jeweils gegenüberliegenden Enden ETS der Trägerstruktur TS jeweilige Halterungen HL aufweist, an welchen der Nutzer die Vorrichtung V greifen kann. Hierdurch kann ein Nutzer in besonders einfacher Art und Weise, wie in Fig. 6 dargestellt, die Rückhalteelemente R in die jeweiligen Vertiefungen VT der Platte P einbringen und so bei Vorsehen mehrerer Rückhalteelemente R in einer einzelnen Vorrichtung V das gleichzeitige Einbringen der Rückhalteelemente R in die Vertiefungen VT ermöglichen. Solche Halterungen HL sind auch in der Fig. 3 detailliert dargestellt als auch in der Fig. 2 gekennzeichnet.

Jede der Halterungen HL weist an ihrer jeweiligen Unterseite HLU, siehe Fig. 3 und Fig. 4, wenigstens ein Beabstandungselement B auf, sodass bei Auflegen der Trägerstruktur TS auf die Oberseite OSP der Multi-Well-Platte P die Beabstandungselemente B ein Beabstanden einer Unterseite UTS der Trägerstruktur TS von der Oberseite OSP der Multi-Well-Platte P bewirken. Dieses kann auch in der Fig. 11a, b oder c sehr gut erkannt werden, da dort ersichtlich wird, dass ein Beabstandungselement B einen Abstand AS zwischen der Unterseite UTS der Trägerstruktur und der Oberseite OSP der Platte herbeiführt. Ein solcher Abstand beträgt vorzugsweise mindestens 2 mm um einen Transport von Flüssigkeiten aufgrund eines Kapillareffektes zwischen der Unterseite UTS der Trägerstruktur und der Oberseite OSP der Platte aufgrund von Kapillarkräften von einer Vertiefung in eine andere Vertiefung zu minimieren.

Die Fig. 1 und 5 zeigen gemeinsam, dass die Vorrichtung V vorzugsweise mehrere, entlang einer geraden Linie angeordnete Öffnungen OET und dazu korrespondierende Rückhalteelemente R aufweist. Vorzugsweise sind diese Rückhalteelemente R in regelmäßigen, äquidistanten Abständen AD zueinander angeordnet.

In der Fig. 3 sind zwei Rückhalteelemente RE1, RE2 in einem mittleren Bereich der Vorrichtung V gekennzeichnet. Die Vorrichtung V weist ferner zwischen den Rückhalteelementen RE1, RE2 an der Unterseite UTS der Trägerstruktur, wie auch in der Fig. gezeigt, ein Beabstandungselement B2 auf, welches bei Auflegen der Trägerstruktur TS auf die Oberseite OSP der Multi-Well-Platte P ein Beabstanden der Unterseite UTS der Trägerstruktur TS von der Oberseite OSP der Multi-Well-Platte P bewirkt.

Hierdurch wird eine Beabstandung zwischen der Unterseite UTS der Trägerstruktur TS und Oberseite OSP der Platte P auch in einem weiteren, mittleren Bereich der Vorrichtung V unterstützt, da bei Vorhandensein mehrerer Rückhalteelemente R in einer geraden Linie eine Biegefähigkeit der Vorrichtung V sonst zu einer Kontaktierung der Unterseite UTS mit der Trägerstruktur TS bzw. einer Reduktion eines Abstandes zwischen der Unterseite UTS der Trägerstruktur TS und der Oberseite OSP der Platte P führen könnte. Auch hierdurch wird ein Übertreten von Flüssigkeit aus einer Vertiefung in eine andere Vertiefung aufgrund von Kapillarkräften zwischen der Unterseite UTS der Trägerstruktur TS und der Oberseite OSP der Platte P minimiert.

Aus der Fig. 5 wird ersichtlich, dass eine jeweilige Öffnung OET einen äußeren Rand RD aufweist. Die Stege ST eines Rückhalteelementes sind rotationssymmetrisch entlang des äußeren Randes RD einer jeweiligen Öffnung OET um die mittlere Symmetrieachse SY des Rückhalteelementes R angeordnet.

In einem linken Bereich der Fig. 5 sind erste Randabschnitte RD1 eingezeichnet, an welchen die Stege ST angeordnet sind. Ferner gibt es zweite Randabschnitte RD2, an welchen sich die nach unten hin erstreckenden Öffnungen SOE zwischen den Stegen ST befinden bzw. an welchen diese angeordnet sind.

Aus der Fig. 5 wird also ersichtlich, dass zueinander, nächstliegende benachbarte Randabschnitte RDX, RDY benachbarter Öffnungen der Trägerstruktur TS jeweils zweite Randabschnitte RD2 sind. Dieses sind also Randabschnitte RD2 mit sich nach unten hin erstreckenden Öffnungen SOE zwischen den Stegen ST. Hierdurch wird ein Übertreten von Flüssigkeit aus einer ersten Vertiefungen in eine andere weiter minimiert, da Flüssigkeit, welche aufgrund von Kapillarkräften zwischen einem Steg ST und einer Innenseite IS einer Vertiefung VT hoch bis zu der Oberseite OSP der Multi-Well-Platte tritt dann nicht den kürzesten Weg hin zu der anderen Vertiefung finden kann. Der kürzeste Weg ist zwischen zwei Randabschnitten RD2 de zweiten Art gegeben, auch als beispielhafte Randabschnitte RDX, RDY eingetragen, an welchen eben insbesondere die Öffnungen SOE angeordnet sind. An diesen Randabschnitten RDX, RDY sind nicht die Stege ST angeordnet.

Vorgeschlagen wird ferner ein Kit K, wie in Fig. 6 und Fig. 7 eingetragen, welches eine Multi-Well-Platte P und eine vorgeschlagene Vorrichtung V aufweist.

Die Fig. 10 zeigt, dass bei Einsetzen der Vorrichtung V in die Multi-Well-Platte P zwischen einer Unterseite U der Vorrichtung mit einer Bodenseite BS einer Vertiefung VT eine Lücke LC1 von vorzugsweise mindestens 1 mm verbleibt. Hierdurch wird es erreicht, dass das Membranelement M genügend Bewegungsfreiheit während des Eluierens bzw. des Lösens der getrockneten Blutbestandteile zur Verfügung hat.

Wie es ferner in der Fig. 10 dargestellt ist, verbleibt vorzugsweise bei Einsetzen der Vorrichtung V in die Multi-Well-Platte P zwischen einem Steg ST und einer Innenseite IS eine Vertiefung VT eine Lücke LC2 von maximal 0,9 mm, bevorzugt 0,8 mm, sonst bevorzugt 0,7 mm, ganz besonders bevorzugt 0,6 mm.

Da beispielsweise ein Membranelement M eine Dicke von vorzugsweise 0,96 mm aufweist kann durch Wahl der hier vorgeschlagenen Dimensionierungen der Lücke LC2 verhindert werden, dass das Membranelement M zwischen Steg ST und Innenseite IS der Vertiefung VT aufgrund einer Auftriebskraft aufsteigt.

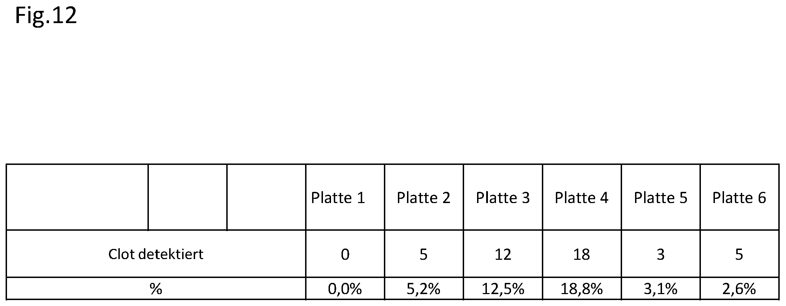

Die Figur 12 zeigt Ergebnisse zur Aspiration von Flüssigkeiten aus Vertiefungen von Multi-Well-Platten für die Fälle, dass entweder kein Membranelement in einer Vertiefung vorhanden war (Platte 1) oder aber dass jeweils ein Membranelement in einer jeweiligen Vertiefung vorhanden war (Platte 2 bis Platte 6). Alle diese Aspirationsversuche wurden ohne die vorgeschlagene Vorrichtung zum Niederhalten von Membranelementen durchgeführt. Alle hier verwendeten Platten, Platte 1 bis Platte 6, wiesen jeweils 96 Wells auf und waren vom Typ "96-Well-Nunc-Platten, F-Bottom, unbeschichtet".

Bei jeder Platte wurden 96 Aspirationen für jeweilige Vertiefungen durchgeführt. Bei der Platte 1 wurde bei 0 Aspirationsvorgängen eine Verstopfung der Nadel bzw. ein Clotereignis detektiert. Bei der Platte 2 wurden bei 5 Aspirationsvorgängen von insgesamt 96 Aspirationsvorgängen Verstopfungen der Nadel bzw. ein Clotereignis detektiert. Bei der Platte 3 war dies bei 12 Aspirationsvorgängen der Fall. Bei der Platte 4 war dies bei 18 Aspirationsvorgängen der Fall. Bei der Platte 5 war dies bei 3 Aspirationsvorgängen der Fall. Bei der Platte 6 war dies bei 5 Aspirationsvorgängen der Fall. Die Ergebnisse aus der Figur 11 zeigen klar den Bedarf des Niederhaltens von Membranelementen um Verstopfungen einer Aspirationsnadel bzw. um Clotereignisse zu vermeiden. Dieses Vermeiden einer Verstopfung einer Aspirationsnadel ist insbesondere deshalb vorteilhaft, da eine Aspiration eines falschen Flüssigkeitsvolumens zu falschen Prozessierungsergebnissen beim Nachweis des Analyten führen kann.

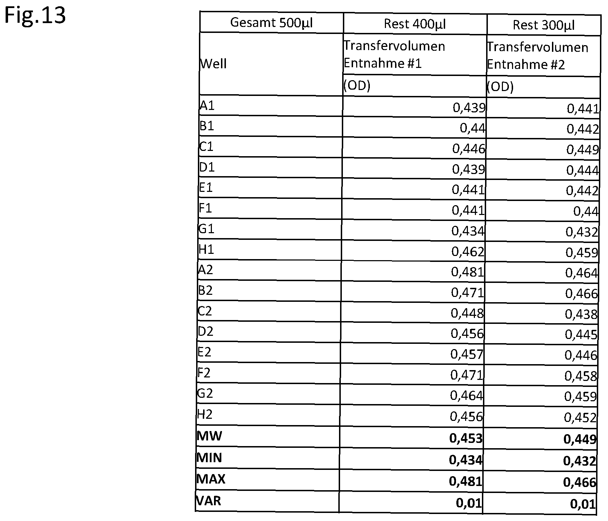

Die Figur 13 zeigt Experimentalergebnisse bei einer Aspiration aus 16 verschiedenen Vertiefungen bzw. 16 verschiedenen Wells mit den Bezeichnungen A1 bis H2 einer Multi-Well-Platte des Typs "Riplate medio 1ml" des Herstellers Ritter. In all diesen Vertiefungen bzw. Wells A1 bis H2 waren jeweils zwei Membranelemente vorhanden sowie 500 ml Flüssigkeit in Form von "Buffer Blue". Die Multi-Well-Platte wurde zunächst in einem Brutschrank zum Inkubieren verwahrt und dann anschließend auf einem Rotationsschüttler geschüttelt. Es wurde dann eine vorgeschlagene Vorrichtung zum Niederhalten bzw. Rückhalten von Membranelementen in die Vertiefungen der Multi-Well-Platte eingesetzt. Es erfolgte dann eine Aspiration aus den jeweiligen Vertiefungen A1 bis H2 mittels des Produktes "EUROLabWorkstation ELISA" der EUROIMMUN Medizinische Labordiagnostika AG. Aus jedem Well wurde in einem ersten Entnahmeschritt zunächst eine Menge von 100 µl aspiriert, sodass ein Rest von 400 µl verblieb. In einem zweiten Entnahmeschritt wurde dann wiederum eine Menge von 100 µl aspiriert, sodass ein Restvolumen von 300 µl verblieb. Jedes der aspirierten Volumina wurde dann in ein separates Behältnis überführt und in dem Verhältnis die optische Dichte (OD) des überführten Volumens gemessen.

Für eine jeweilige Vertiefung A1 bis H2 sind die jeweils gemessenen optischen Dichten in der mittleren Spalte für den ersten Entnahmeschritt aufgeführt und in der rechten Spalte für den zweiten Entnahmeschritt. Die optische Dichte ist ein Maß bzw. ein Indikator für die aspirierte und überführte Probenmenge bzw. das aspirierte und überführte Volumen. Die Ergebnisse zeigen, dass die gemessenen optischen Dichten für beide Schritte über alle Volumina sehr ähnliche Werte aufweisen und sich eine Varianz von lediglich 0,01 ergibt. Die sehr geringe Varianz dieser optischen Dichte weist darauf hin, dass für alle Wells A1 bis H2 auch in den jeweiligen Entnahmeschritten 1 und 2 fast identische Probenvolumina aspiriert und überführt wurden. Dieses zeigt, dass in keinem Fall eine maßgebliche Verstopfung der Aspirationsnadel durch ein Membranelement vorkam.

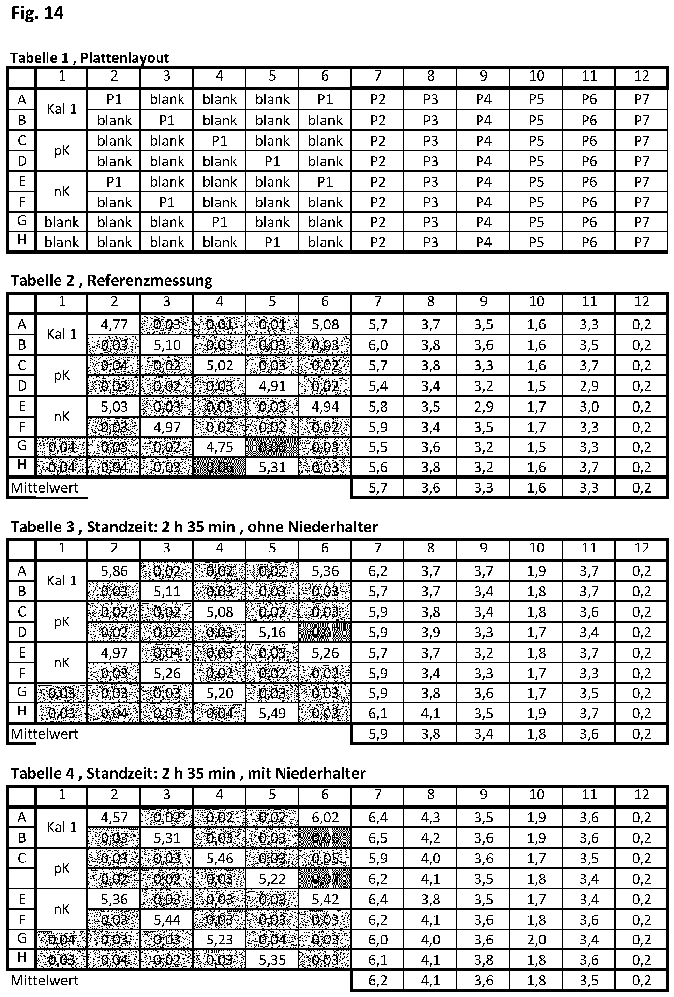

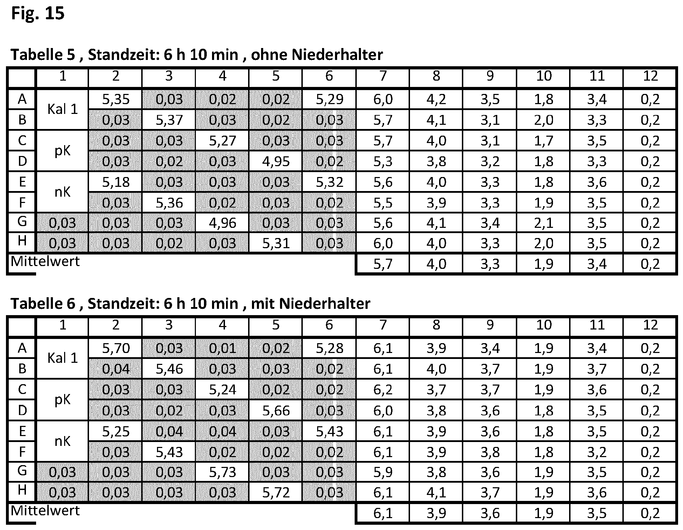

Die Figuren 14 und 15 zeigen in den Tabellen 1 bis Tabelle 6 weitere Ergebnisse bezüglich eines möglichen Übertretens von Probenflüssigkeit von einer Vertiefung in eine andere Vertiefung. Die Tabelle 1 zeigt ein Layout einer Multi-Well-Platte mit Zeilen A bis H und Spalten 1 bis 12, wie es für eine 96er Multi-Well-Platte üblich ist. Es wurde jeweils Kalibratorflüssigkeit in die Vertiefungen A1 und B1 eingefüllt. In weitere zwei Vertiefungen C1, D1 wurden Positivkontrollen eingefüllt. In zwei weitere Vertiefungen E1, F1 wurden Negativkontrollen eingefüllt. In die Vertiefungen G1 und H1 wurde jeweils nur sogenannte Pufferflüssigkeit eingefüllt. In weitere unterschiedliche Vertiefungen wurden Proben mit dem Index P1 bis P7 eingefüllt. Vertiefungen, für die die Angabe "blank" gegeben ist, wurden nur jeweils mit Pufferflüssigkeit aufgefüllt.

Es wurde dann für eine jede der jeweiligen Vertiefungen eine Referenzmessung der optischen Dichte durchgeführt, wobei die in der Tabelle 2 gezeigten Messwerte eine relative optische Dichte als einen Quotient der optischen Dichte der jeweiligen Probe der jeweiligen Vertiefung geteilt durch die optische Dichte der Kalibratorflüssigkeit aus den Vertiefungen A1 bzw. B1 wiedergibt.

Es geht aus der Tabelle 2 hervor, dass Vertiefungen mit tatsächlichen Proben mit Index P1 bis P7 jeweils direkt nach dem Befüllen Werte der relativen optischen Dichte aufweisen, welche deutlich höher sind als die jener Vertiefungen, welche nur Pufferflüssigkeit aufweisen.

Hellgrau markierte Messwerte weisen hierbei einen optischen Dichtequotient von weniger als 0,05 auf. Dunkelgrau markierte Messwerte weisen hierbei einen Dichtequotient von weniger als 0,2 auf.

Die Tabelle 3 aus der Figur 14 zeigt gemessene Quotienten von relativen optischen Dichten für die jeweiligen Vertiefungen nach einer Standzeit von 2 Stunden und 35 Minuten, ohne dass vorgeschlagene Vorrichtung zum Niederhalten bzw. Rückhalten von Membranelementen eingesetzt wurde. Es konnte also keine Verschleppung von Flüssigkeit aus einer Vertiefung zu einer anderen durch eine solche Vorrichtung bewirkt werden. Die Tabelle 4 zeigt entsprechende relative optische Dichten für die jeweiligen Vertiefungen in dem Fall, dass für eine Standzeit von 2 Stunden und 35 Minuten die vorgeschlagene Vorrichtung in die jeweiligen Vertiefungen eingesetzt war. Ein Vergleich der Messergebnisse aus Tabelle 3 und Tabelle 4 zeigt deutlich, dass durch die vorgeschlagene Vorrichtung keine maßgebliche Verschleppung von Probenflüssigkeit aus einer Vertiefung in eine andere bewirkt werden konnte. Ferner stiegen Werte der relativen der optischen Dichte für Vertiefungen, welche nur Probenflüssigkeit aufwiesen, in keinem der Fälle merklich an, weder mit oder ohne Verwendung der vorgeschlagenen Vorrichtung.

Die Figur 15 zeigt entsprechende Ergebnisse in Tabelle 5 für eine Standzeit von 6 Stunden und 10 Minuten ohne die Verwendung der vorgeschlagenen Vorrichtung als auch in der Tabelle 6 unter Verwendung der vorgeschlagenen Vorrichtung. Auch hier kann keine maßgebliche Verschleppung von Flüssigkeit auf einer Vertiefung in eine andere beobachtet werden. Dies unterstreicht die Verwendbarkeit der vorgeschlagenen Vorrichtung.

## Patentansprüche

1. Vorrichtung (V) zum Niederhalten von jeweiligen Membranelementen (M) in jeweiligen Vertiefungen (VT) einer Multi-Well-Platte (P),
wobei die Multi-Well-Platte (P) an ihrer Oberseite (OSP) jeweilige Öffnungen (EOP) der jeweiligen Vertiefungen (VT) aufweist und wobei die jeweiligen Vertiefungen (VT) jeweils zur Aufnahme einer jeweiligen flüssigen Lösung (FL) und zur Aufnahme der jeweiligen Membranelemente (M) geeignet sind,
wobei die Vorrichtung (V) an ihrem oberen Bereich (OB) eine Trägerstruktur (TS) mit jeweiligen Öffnungen (OET) aufweist sowie jeweilige korrespondierende sich von den jeweiligen Öffnungen (OET) der Trägerstruktur (TS) nach unten hin erstreckenden Rückhalteelementen (R), sodass bei Auflegen der Trägerstruktur (TS) auf die Oberseite (OSP) der Multi-Well-Platte (P) jeweilige der Rückhalteelemente (R) in jeweilige der Vertiefungen (VT) hineinragen,
wobei ein jeweiliges der Rückhalteelemente (R) mehrere sich von der Trägerstruktur (TS) her nach unten hin erstreckende Stege (ST) aufweist, deren Enden (E) an einer Unterseite (U) der Vorrichtung (V) mittels eines insbesondere ringartigen Verbindungselementes (VE), welches eine mittlere Öffnung (MO) aufweist, miteinander verbunden sind,
wobei ferner ein jeweiliges der Rückhalteelemente (R) zwischen den Stegen (ST) mehrere Öffnungen (SOE) aufweist, welche sich von der Trägerstruktur (TS) her bis hin zu dem Verbindungselement (VE) durchgehend erstrecken und welche ferner sich bei Auflegen der Trägerstruktur (TS) auf die Oberseite (OSP) der Multi-Well-Platte (P) von der Oberseite (OSP) der Multi-Well-Platte (P) her bis hin zu dem Verbindungselement (VE) durchgehend erstrecken,
wobei die Stege (ST) symmetrisch um eine mittlere Symmetrieachse des Rückhalteelementes (R) angeordnet sind
wobei die Stege (ST) von oben her nach unten hin konisch aufeinander zulaufen
wobei die Stege (ST) eines Rückhalteelementes (R) auf einem äußeren Rand (RD) der jeweiligen Öffnung (OET) der Trägerstruktur (TS) rotationssymmetrisch um die mittlere Symmetrieachse (SY) des Rückhalteelementes (R) angeordnet sind,
wobei an ersten Randabschnitten (RD1) des Randes die Stege angeordnet sind
**dadurch gekennzeichnet, dass**
an zweiten Randabschnitten (RD2) des Randes (RD) die sich nach unten hin erstreckenden Öffnungen (SOE) zwischen den Stegen (ST) angeordnet sind, sodass zueinander nächstliegende, benachbarten Randabschnitte (RDX, RDY) benachbarter Öffnungen der Trägerstruktur (TS) jeweils zweite Randabschnitte (RD2) sind.

2. Vorrichtung nach Anspruch 1,
wobei jeweilige Unterseiten (US) der Verbindungselemente (VE) eine Unterseite (U) der Vorrichtung (V) bilden
und wobei die Unterseite (U) der Vorrichtung (V) in einer Ebene (E2) liegt bzw. eine Ebene (E2) bildet, welche parallel zu einer Ebene (E1) der Trägerstruktur (TS) ist.

3. Vorrichtung nach Anspruch 1,
wobei ein jeweiliges der Rückhalteelemente (R) so ausgebildet ist, dass entlang einer mittleren Symmetrieachse (SY) des Rückhalteelementes (R) von oben her durch die jeweils korrespondierende Öffnung (OET) an der Trägerstruktur (TS) eine Pipettiernadel (PN) nach unten hin bis hin zu dem insbesondere ringartigen Verbindungselement (VE) eingeführt werden kann.

4. Vorrichtung nach Anspruch 1,
wobei die Vorrichtung (V) an jeweils gegenüberliegenden Enden (ETS) der Trägerstruktur (TS) jeweilige Halterungen (HL) aufweist, welche durch einen Nutzer gegriffen werden können.

5. Vorrichtung nach Anspruch 4,
wobei jede der Halterungen (HL) an ihrer jeweiligen Unterseite (HLU) wenigstens ein Beabstandungselement (B) aufweist, sodass bei Auflegen der Vorrichtung (V) auf die Oberseite (OSP) der Multi-Well-Platte (P) die Beabstandungselemente (B) ein Beabstanden einer Unterseite (UTS) der Trägerstruktur (TS) von der Oberseite der Multi-Well-Platte (P) bewirken.

6. Vorrichtung nach Anspruch 5,
wobei die Vorrichtung (V) mehrere, entlang einer Linie (GL) angeordnete Öffnungen (OEP) und dazu korrespondierende Rückhalteelemente (R) aufweist,
und wobei die Vorrichtung (V) ferner zwischen zwei Öffnungen (OEPA, OEPB) an der Unterseite (UTS) der Trägerstruktur (TS) ein weiteres Beabstandungselement (B2) aufweist, welches bei Auflegen der Vorrichtung auf die Oberseite (OSP) der Multi-Well-Platte (P) ein Beabstanden der Unterseite (UTS) der Trägerstruktur (TS) von der Oberseite (OPS) der Multi-Well-Platte (P) bewirkt.

7. Kit (K) aufweisend eine Multi-Well-Platte (P) und eine Vorrichtung (V) nach einem der vorhergehenden Ansprüche.

8. Kit nach Anspruch 7,
wobei bei Einsetzen der Vorrichtung (V) in die Multi-Well-Platte (P) zwischen einer Unterseite (U) der Vorrichtung (V) und einer Bodenseite (BS) einer Vertiefung (VT) eine Lücke (LC) von mindestens 1 mm verbleibt.

9. Kit (K) nach Anspruch 7,
wobei bei Einsetzen der Vorrichtung (V) in die Multi-Well-Platte (P) zwischen einem Steg (ST) und einer Innenseite (IS) einer Vertiefung (VT) eine Lücke (LC2) von maximal 0,9 mm verbleibt.

10. Verfahren zum Nachweis eines Analyten in getrockneten Blutbestandteilen umfassend die Schritte
- Bereitstellen eines Membranelementes, welches die getrockneten Blutbestandteile aufweist,
- Einbringen des Membranelementes in eine Vertiefung einer Multi-Well-Platte,
- Einbringen einer Flüssigkeit, die geeignet ist, um den Analyten aus der eingetrockneten Blutprobe aufzunehmen, in die Vertiefung,
- Ineinanderfügen der Multi-Well-Platte mit einer Vorrichtung nach einem der Ansprüche 1 bis 6, so dass das Membranelement durch die Vorrichtung in einem unteren Bereich der Vertiefung unterhalb der Flüssigkeitsoberfläche positioniert wird,
- Einführen einer Pipettiernadel in die Vertiefung und Aspirieren zumindest eines Teilvolumens der Flüssigkeit,
- Überführen des Teilvolumens der Flüssigkeit in ein Aufnahmegefäß,
- Nachweisen des Analyten in dem Teilvolumen der Flüssigkeit.

## Claims

1. Apparatus (V) for holding down respective membrane elements (M) in respective wells (VT) of a multiwell plate (P),
wherein the multiwell plate (P) has, at its top side (OSP), respective openings (EOP) of the respective wells (VT) and wherein the respective wells (VT) are each suitable for accommodation of a respective liquid solution (FL) and for accommodation of the respective membrane elements (M),
wherein the apparatus (V) has, at its upper region (OB), a support structure (TS) having respective openings (OET), and respective corresponding retention elements (R) which extend downwards from the respective openings (OET) of the support structure (TS), so that respective retention elements (R) protrude into respective wells (VT) when the support structure (TS) has been placed onto the top side (OSP) of the multiwell plate (P),
wherein a respective retention element (R) has multiple webs (ST) which extend downwards from the support structure (TS) and the ends (E) of which are connected to one another at a bottom side (U) of the apparatus (V) by means of an in particular annular connection element (VE) which has a central opening (MO),
wherein furthermore a respective retention element (R) has multiple openings (SOE) between the webs (ST), which openings extend continuously from the support structure (TS) right up to the connection element (VE) and which openings furthermore extend continuously from the top side (OSP) of the multiwell plate (P) right up to the connection element (VE) when the support structure (TS) has been placed onto the top side (OSP) of the multiwell plate (P),
wherein the webs (ST) are arranged symmetrically around a central axis of symmetry of the retention element (R),
and wherein the webs (ST) conically taper downwards from above,
wherein the webs (ST) of a retention element (R) are arranged rotationally symmetrically around the central axis of symmetry (SY) of the retention element (R) on an outer edge (RD) of the respective opening (OET) of the support structure (TS),
wherein the webs are arranged at first edge sections (RD1) of the edge
**characterized in that** the downwardly extending openings (SOE) between the webs (ST) are arranged at second edge sections (RD2) of the edge (RD), such that mutually closest, adjacent edge sections (RDX, RDY) of adjacent openings of the support structure (TS) are respectively second edge sections (RD2).

2. Apparatus according to Claim 1,
wherein respective bottom sides (US) of the connection elements (VE) form a bottom side (U) of the apparatus (V)
and wherein the bottom side (U) of the apparatus (V) lies in a plane (E2) or forms a plane (E2) which is parallel to a plane (E1) of the support structure (TS).

3. Apparatus according to Claim 1,
wherein a respective retention element (R) is designed such that a pipetting needle (PN) can be inserted downwards from above through the respectively corresponding opening (OET) at the support structure (TS) along a central axis of symmetry (SY) of the retention element (R) right up to the in particular annular connection element (VE).

4. Apparatus according to Claim 1,
wherein the apparatus (V) has, at respectively opposing ends (ETS) of the support structure (TS), respective mounts (HL) which can be gripped by a user.

5. Apparatus according to Claim 4,
wherein each of the mounts (HL) has, at its respective bottom side (HLU), at least one spacing element (B), so that the spacing elements (B) bring about spacing of a bottom side (UTS) of the support structure (TS) from the top side of the multiwell plate (P) when the apparatus (V) has been placed onto the top side (OSP) of the multiwell plate (P).

6. Apparatus according to Claim 5,
wherein the apparatus (V) has multiple openings (OEP) arranged along a straight line (GL) and corresponding retention elements (R),
and wherein the apparatus (V) has furthermore, between two openings (OEPA, OEPB) at the bottom side (UTS) of the support structure (TS), a further spacing element (B2) which brings about spacing of the bottom side (UTS) of the support structure (TS) from the top side (OPS) of the multiwell plate (P) when the apparatus has been placed onto the top side (OSP) of the multiwell plate (P).

7. Kit (K) comprising a multiwell plate (P) and an apparatus (V) according to any of the preceding claims.

8. Kit according to Claim 7,
wherein a gap (LC) of at least 1 mm remains between a bottom side (U) of the apparatus (V) and a base side (BS) of a well (VT) when the apparatus (V) has been inserted into the multiwell plate (P).

9. Kit (K) according to Claim 7,
wherein a gap (LC2) of not more than 0.9 mm remains between a web (ST) and an inner side (IS) of a well (VT) when the apparatus (V) has been inserted into the multiwell plate (P).

10. Method for detecting an analyte in dried blood constituents, comprising the steps of
- providing a membrane element which comprises the dried blood constituents,
- introducing the membrane element into a well of a multiwell plate,
- introducing into the well a liquid suitable for taking up the analyte from the dried blood sample,
- fitting the multiwell plate together with an apparatus according to any of Claims 1 to 6 so that the membrane element is positioned in a lower region of the well below the liquid surface by the apparatus,
- inserting a pipetting needle into the well and aspirating at least a partial volume of the liquid,
- transferring the partial volume of the liquid into an accommodation vessel,
- detecting the analyte in the partial volume of the liquid.

## Revendications

1. Dispositif (V) pour maintenir des éléments membranaires respectifs (M) dans des cavités respectives (VT) d'une plaque multi-puits (P),
dans lequel la plaque multi-puits (P) présente sur son côté supérieur (OSP) des ouvertures respectives (EOP) des cavités respectives (VT) et dans lequel les cavités respectives (VT) sont chacune appropriées pour recevoir une solution liquide respective (FL) et pour recevoir les éléments membranaires respectifs (M),
dans lequel le dispositif (V) présente, au niveau de sa zone supérieure (OB), une structure de support (TS) avec des ouvertures respectives (OET) ainsi que des éléments de retenue respectifs correspondants (R) s'étendant vers le bas à partir des ouvertures respectives (OET) de la structure de support (TS), de telle sorte que, lorsque la structure de support (TS) est placée sur le côté supérieur (OSP) de la plaque multi-puits (P), des éléments de retenue respectifs (R) font saillie dans des cavités respectives (VT),
dans lequel chacun des éléments de retenue (R) présente plusieurs entretoises (ST) s'étendant vers le bas à partir de la structure de support (TS), dont les extrémités (E) sont reliées entre elles sur un côté inférieur (U) du dispositif (V) au moyen d'un élément de liaison (VE), notamment de type annulaire, qui présente une ouverture centrale (MO),
dans lequel, en outre, chacun des éléments de retenue (R) présente, entre les entretoises (ST), plusieurs ouvertures (SOE) qui s'étendent de manière continue depuis la structure de support (TS) jusqu'à l'élément de liaison (VE) et qui, en outre, lorsque la structure de support (TS) est placée sur le côté supérieur (OSP) de la plaque multi-puits (P), s'étendent de manière continue depuis le côté supérieur (OSP) de la plaque multi-puits (P) jusqu'à l'élément de liaison (VE),
dans lequel les entretoises (ST) sont agencées symétriquement autour d'un axe de symétrie central de l'élément de retenue (R)
dans lequel les entretoises (ST) s'effilent les unes vers les autres du haut vers le bas dans lequel les entretoises (ST) d'un élément de retenue (R) sont agencées sur un bord extérieur (RD) de l'ouverture respective (OET) de la structure de support (TS) avec une symétrie de rotation autour de l'axe de symétrie central (SY) de l'élément de retenue (R),
les entretoises étant agencées sur des premières sections de bord (RD1) du bord,
**caractérisé en ce que** sur des deuxièmes sections de bord (RD2) du bord (RD), les ouvertures (SOE) s'étendant vers le bas sont agencées entre les entretoises (ST), de telle sorte que des sections de bord (RDX, RDY) voisines les plus proches les unes des autres d'ouvertures voisines de la structure de support (TS) sont respectivement des deuxièmes sections de bord (RD2).

2. Dispositif selon la revendication 1,
dans lequel les côtés inférieurs respectifs (US) des éléments de liaison (VE) forment un côté inférieur (U) du dispositif (V)
et dans lequel le côté inférieur (U) du dispositif (V) se situe dans un plan (E2) ou forme un plan (E2) qui est parallèle à un plan (E1) de la structure de support (TS).

3. Dispositif selon la revendication 1,
dans lequel chacun des éléments de retenue (R) est réalisé de telle sorte que, le long d'un axe de symétrie central (SY) de l'élément de retenue (R), une aiguille de pipetage (PN) peut être insérée à partir du haut à travers l'ouverture correspondante respective (OET) sur la structure de support (TS) vers le bas jusqu'à l'élément de liaison (VE) notamment de type annulaire.

4. Dispositif selon la revendication 1,
dans lequel le dispositif (V) présente, aux extrémités opposées respectives (ETS) de la structure de support (TS), des fixations respectives (HL) qui peuvent être saisies par un utilisateur.

5. Dispositif selon la revendication 4,
dans lequel chacune des fixations (HL) présente au moins un élément d'espacement (B) sur son côté inférieur respectif (HLU), de telle sorte que lorsque le dispositif (V) est placé sur le côté supérieur (OSP) de la plaque multi-puits (P), les éléments d'espacement (B) provoquent un espacement d'un côté inférieur (UTS) de la structure de support (TS) par rapport au côté supérieur de la plaque multi-puits (P).

6. Dispositif selon la revendication 5,
dans lequel le dispositif (V) présente plusieurs ouvertures (OEP) agencées le long d'une ligne (GL) et des éléments de retenue (R) correspondant à celles-ci,
et dans lequel le dispositif (V) présente en outre, entre deux ouvertures (OEPA, OEPB) sur le côté inférieur (UTS) de la structure de support (TS), un autre élément d'espacement (B2) avec lequel, lorsque le dispositif est placé sur le côté supérieur (OSP) de la plaque multi-puits (P), il provoque un espacement du côté inférieur (UTS) de la structure de support (TS) par rapport au côté supérieur (OPS) de la plaque multi-puits (P).

7. Kit (K) présentant une plaque multi-puits (P) et un dispositif (V) selon l'une quelconque des revendications précédentes.

8. Kit selon la revendication 7,
dans lequel, lorsque le dispositif (V) est inséré dans la plaque multi-puits (P), il reste un espace (LC) d'au moins 1 mm entre un côté inférieur (U) du dispositif (V) et un côté inférieur (BS) d'une cavité (VT).

9. Kit (K) selon la revendication 7,
dans lequel, lorsque le dispositif (V) est inséré dans la plaque multi-puits P, il reste un espace (LC2) de 0,9 mm maximum entre une entretoise (ST) et un côté intérieur (IS) d'une cavité (VT).

10. Procédé de détection d'un analyte dans des composants sanguins séchés, comprenant les étapes suivantes
- la fourniture d'un élément membranaire présentant les composants sanguins séchés,
- l'introduction de l'élément membranaire dans une cavité d'une plaque multi-puits,
- l'introduction dans la cavité d'un liquide approprié pour absorber l'analyte de l'échantillon de sang séché,
- l'emboîtement de la plaque multi-puits avec un dispositif selon l'une quelconque des revendications 1 à 6, de telle sorte que l'élément membranaire est positionné par le dispositif dans une zone inférieure de la cavité en dessous de la surface du liquide,
- l'insertion d'une aiguille de pipetage dans la cavité et l'aspiration d'au moins un volume partiel du liquide,
- le transfert du volume partiel de liquide dans un récipient de réception,
- la détection de l'analyte dans le volume partiel du liquide
